(19) **Europäisches Patentamt / European Patent Office / Office européen des brevets**

(11) **EP 3 342 410 A1**

(12) **EUROPEAN PATENT APPLICATION**
published in accordance with Art. 153(4) EPC

(43) Date of publication:
**04.07.2018 Bulletin 2018/27**

(21) Application number: **16841644.4**

(22) Date of filing: **25.08.2016**

(51) Int Cl.:
*A61K 31/436* (2006.01)   *A61P 29/00* (2006.01)
*A61P 35/00* (2006.01)   *A61P 37/02* (2006.01)
*A61P 37/06* (2006.01)

(86) International application number:
**PCT/JP2016/074781**

(87) International publication number:
**WO 2017/038612 (09.03.2017 Gazette 2017/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **28.08.2015 JP 2015169075**

(71) Applicant: **Nippon Kayaku Kabushiki Kaisha
Tokyo 100-0005 (JP)**

(72) Inventor: **KAWAMURA Dai
Tokyo 115-8588 (JP)**

(74) Representative: **Godemeyer Blum Lenze
Patentanwälte
Partnerschaft mbB - werkpatent
An den Gärten 7
51491 Overath (DE)**

(54) **PHARMACEUTICAL COMPOSITION CONTAINING RAPAMYCIN OR DERIVATIVE THEREOF**

(57)    It is an object of the present invention to provide a pharmaceutical composition, which can suppress a reduction in the content of an active ingredient caused by oxidation or decomposition of rapamycin or a derivative thereof, can ensure long-term stability, and has high safety. In the present invention, the inventors have found that a tocopherol derivative and an ascorbic acid fatty acid ester are used in combination in a pharmaceutical formulation comprising rapamycin or a derivative thereof, so that oxidation or decomposition of the rapamycin or a derivative thereof can be suppressed, thereby completing the present invention. The rapamycin or a derivative thereof, and the tocopherol derivative and the ascorbic acid fatty acid ester are preferably in the form of a solid mixture produced by preparing a solution containing these components and then removing the solvent from the solution.

EP 3 342 410 A1

**Description**

Technical Field

The present invention relates to a pharmaceutical formulation composition comprising rapamycin or a derivative thereof, the stability of which has been improved. Since rapamycin or a derivative thereof is extremely instable to oxygen, light and moisture, it is problematic in terms of preservation stability. The present invention is a technique relating to a pharmaceutical formulation composition, in which the preservation stability of such a compound has been improved by a stabilizer having excellent safety with consideration to *in vivo* administration.

Background Art

It has been known that rapamycin (sirolimus) is a macrolide antibiotic discovered from the metabolite of actinomyces and has an immunosuppressive action. Rapamycin has an action to inhibit a mammalian rapamycin target protein (mammalian target of rapamycin; mTOR) that regulates cell division, cell growth, survival, etc. This mTOR is a main serine-threonine kinase, which regulates the synthesis of proteins by stimulation with growth factors, nutrients, etc., and the mTOR has been known to regulate the growth, proliferation and survival of cells, and angiogenesis. Hence, the mTOR inhibitory action of rapamycin has been focused, and the synthesis of a derivative thereof has been attempted. As a result, everolimus and temsirolimus have been discovered as antitumor agents.

A pharmaceutical formulation used to provide a pharmaceutical product comprising rapamycin or a derivative thereof has been reported. Patent Literature 1 discloses that a solution containing a mixture of rapamycin, hydroxypropylmethyl cellulose, lactose and the like is prepared, the solvent is then distilled away from the solution, and the obtained solid dispersion is then formulated. In addition, Patent Literature 2 discloses a tablet comprising everolimus used as a rapamycin, crospovidone used as a disintegrator, colloidal silicon dioxide, and lactose.

Rapamycin or a derivative thereof has been known to have physical properties by which it is extremely instable to oxidation. Hence, in general, an antioxidant is added to a pharmaceutical product formulation comprising, as an active ingredient, rapamycin. For instance, to a rapamycin preparation (registered trademark: Rapalimus) and a temsirolimus preparation (registered trademark: Torisel), tocopherol is added. In addition, for everolimus formulations (registered trademark: Afinitor and Certican), a synthetic antioxidant, dibutylhydroxytoluene (BHT) is used.

Regarding a method of stabilizing a rapamycin derivative using an antioxidant, Patent Literature 3 discloses that a mixed solution containing everolimus and BHT as an antioxidant is prepared, and the solvent is then removed from the mixed solution, so as to obtain a stabilized everolimus solid. Examples of the antioxidant used in this publication include BHT, tocopherol and ascorbic acid.

However, it has been reported that BHT used as an antioxidant exhibits carcinogenicity or reproduction toxicity, and thus, this is a chemical substance, the amount used of which is limited. On the other hand, tocopherol has higher safety than BHT, but its antioxidative activity is lower than that of BHT when it is used alone. Thus, the effect of tocopherol to stabilize rapamycin based on its antioxidative action has a certain limit.

As other antioxidants used as additives for pharmaceutical products, there have been known ascorbic acid, and ascorbyl palmitate as a fat-soluble derivative of ascorbic acid. Since these antioxidants also have antioxidative effects that are inferior to those of synthetic antioxidants such as BHT when it is used alone, they are insufficient to be applied to pharmaceutical formulations comprising rapamycin.

Patent Literature 4 discloses that an ethanol solution of everolimus is added to a water-soluble polymer such as hypromellose, and the mixture is then granulated to prepare a solid dispersion, thereby obtaining a stable everolimus composition, without using antioxidants.

Prior Art Literatures

Patent Literatures

Patent Literature 1: JP Patent Publication (Kohyo) No. 11-509223 A (1999)
Patent Literature 2: JP Patent Publication (Kohyo) No. 2005-507897 A
Patent Literature 3: JP Patent Publication (Kohyo) No. 2002-531527 A
Patent Literature 4: International Publication No. WO 2013/022201

Summary of Invention

Object to be Solved by the Invention

It is an object of the present invention to provide a pharmaceutical composition and a pharmaceutical formulation, used in a pharmaceutical formulation comprising rapamycin or a derivative thereof, wherein the pharmaceutical composition can suppress a reduction in the content of an active ingredient caused by oxidation or decomposition of the rapamycin or a derivative thereof, can ensure long-term stability, and has high safety. It is another object of the present invention to provide a method for producing a pharmaceutical composition and a pharmaceutical formulation comprising rapamycin or a derivative thereof, wherein the composition and formulation can ensure long-term stability and has high safety.

Means for Solving the Object

The present inventors have found that, in order to suppress oxidation or decomposition of rapamycin or a derivative thereof in a pharmaceutical formulation comprising the rapamycin or a derivative thereof, a tocopherol derivative that is a natural antioxidant is used as an additive, and further, an ascorbic acid fatty acid ester is used as a synergist, so that synergistic antioxidative effects can be exhibited for a long period of time, thereby completing the present invention. Specifically, the present application includes the inventions according to the following [1] to [9] as features.

[1] A pharmaceutical composition comprising (A) rapamycin or a derivative thereof, and (B) a tocopherol derivative and (C) an ascorbic acid fatty acid ester.
In the present invention, a pharmaceutical composition is allowed to comprise an antioxidant consisting of a combination of (B) the tocopherol derivative and (C) the ascorbic acid fatty acid ester, with respect to the rapamycin or a derivative thereof, so that the composition, in which the stability of the rapamycin or a derivative thereof is improved, can be produced.
[2] The pharmaceutical composition according to the above [1], which is produced by preparing a solution containing (A) rapamycin or a derivative thereof, and (B) a tocopherol derivative and (C) an ascorbic acid fatty acid ester, and then removing the solvent from the solution.
As an aspect of mixing the rapamycin or a derivative thereof with the antioxidant consisting of a combination of (B) the tocopherol derivative and (C) the ascorbic acid fatty acid ester, it is preferable to prepare a solution containing the above-described components (A), (B) and (C), and then to obtain a solid mixture from the solution. Such a solid mixture is a mixture formed by associating the rapamycin or a derivative thereof with the tocopherol derivative and the ascorbic acid fatty acid ester at a molecular level, and thus, it is difficult to explain this solid mixture with a chemical structure, properties, etc. Hence, a more detailed aspect of the pharmaceutical composition according to the present invention, which comprises (A) rapamycin or a derivative thereof and an antioxidant consisting of a combination of (B) a tocopherol derivative and (C) an ascorbic acid fatty acid ester, is appropriately expressed as a solid mixture comprising rapamycin or a derivative thereof, and a tocopherol derivative and an ascorbic acid fatty acid ester, which is specified by the production method according to the above [2], and this pharmaceutical composition is considered to satisfy the requirements regarding the clarity of the invention.
[3] The pharmaceutical composition according to the above [1] or [2], which comprises 0.0001 to 5.0 parts by mass of (B) the tocopherol derivative and 0.001 to 10.0 parts by mass of (C) the ascorbic acid fatty acid ester, based on 1 part by mass of (A) the rapamycin or a derivative thereof, and the mixing ratio between the components (B) and (C), (B) : (C), is 1 : 0.1 to 100.
[4] The pharmaceutical composition according to any one of the above [1] to [3], which further comprises an antioxidant other than (B) the tocopherol derivative and (C) the ascorbic acid fatty acid ester.
[5] The pharmaceutical composition according to any one of the above [1] to [4], which comprises a cellulose derivative and/or sugars.
[6] A pharmaceutical formulation comprising the pharmaceutical composition according to any one of the above [1] to [5].
Moreover, the present invention also includes, as a feature thereof, a method for producing a pharmaceutical composition and a pharmaceutical formulation comprising rapamycin or a derivative thereof, and a tocopherol derivative and an ascorbic acid fatty acid ester.
[7] A method for producing a pharmaceutical composition comprising rapamycin or a derivative thereof, which comprises preparing a solution containing (A) rapamycin or a derivative thereof, and (B) a tocopherol derivative and (C) an ascorbic acid fatty acid ester, and then removing the solvent from the solution.
According to the above-described production method, a solid mixture, in which rapamycin or a derivative thereof is associated with a tocopherol derivative and an ascorbic acid fatty acid ester at a molecular level, can be produced.

Furthermore, when an antioxidant other than (B) the tocopherol derivative and (C) the ascorbic acid fatty acid ester is used, such an antioxidant may be added upon preparation of the solution, or may also be added to the solid mixture.

[8] A method for producing a pharmaceutical formulation comprising rapamycin or a derivative thereof, which comprises preparing a solution containing (A) rapamycin or a derivative thereof, and (B) a tocopherol derivative and (C) an ascorbic acid fatty acid ester, then mixing the solution with a cellulose derivative and/or sugars, and then removing the solvent from the mixture.

[9] A method for producing a pharmaceutical formulation comprising rapamycin or a derivative thereof, which comprises preparing a solution containing (A) rapamycin or a derivative thereof, and (B) a tocopherol derivative and (C) an ascorbic acid fatty acid ester, and then removing the solvent from the prepared solution to obtain a pharmaceutical composition comprising the rapamycin or a derivative thereof, and adding a cellulose derivative and/or sugars to the above-described pharmaceutical composition.

As in the production method according to the above [8] or [9],upon production of a pharmaceutical formulation comprising rapamycin or a derivative thereof, the cellulose derivative and/or sugars used as pharmaceutical additives may be added at the stage of preparing a solid mixture, in which the rapamycin or a derivative thereof is associated with the tocopherol derivative and the ascorbic acid fatty acid ester at a molecular level, or may also be added after preparation of the solid mixture.

Advantageous Effects of Invention

According to the present invention, there can be provided a pharmaceutical composition comprising rapamycin or a derivative thereof, wherein the pharmaceutical composition can suppress a reduction in the content of an active ingredient caused by oxidation or decomposition of the rapamycin or a derivative thereof, can ensure long-term stability, and has high safety. That is to say, long-term stability, which could not been ensured when a highly safe natural antioxidant, tocopherol, was added alone, can be ensured by using an ascorbic acid fatty acid ester as a synergist.

The pharmaceutical composition and pharmaceutical formulation of the present invention are pharmaceutical formulations, which avoid the use of BHT that is problematic in terms of carcinogenicity or reproduction toxicity, and these are pharmaceutical formulations comprising rapamycin or a derivative thereof, which ensure stability and safety.

Embodiments for Carrying out the Invention

The pharmaceutical composition of the present invention is characterized in that it comprises (A) rapamycin or a derivative thereof, and (B) a tocopherol derivative and (C) an ascorbic acid fatty acid ester. The details thereof will be described below.

The present invention comprises, as an active ingredient, (A) rapamycin or a derivative thereof.

Rapamycin (common name: Sirolimus) is a compound having a macrolide skeleton that has been isolated from the metabolite of actinomyces, *Streptomyces Hygroscopicus,* separated from the soil of Easter Island.

The term "rapamycin derivative" means a substance prepared by chemically modifying rapamycin used as a mother core. Examples of the rapamycin derivative include 16-O-substituted rapamycin (see, for example, WO 94/022136), 40-O-substituted rapamycin (see, for example, US 5258389 and WO 94/09010), carboxylic acid ester-substituted rapamycin (see, for example, WO 92/05179), amide-substituted rapamycin (see, for example, US 5118677), fluorine-substituted rapamycin (see, for example, US 5100883), and acetal-substituted rapamycin (see, for example, US 5151413). The rapamycin or a derivative thereof of the present invention is not limited thereto, but the aforementioned rapamycin derivatives can be used as applicable preferred compounds.

The rapamycin derivative is preferably a 40-O-substituted rapamycin derivative, in which the hydroxyl group at position 40 of the cyclohexyl group of rapamycin is substituted with a hydroxyalkyl group, a hydroxyalkoxyalkyl group, an acylaminoalkyl group, an aminoalkyl group, or a hydroxy-substituted acyl group. The rapamycin derivative is more preferably 40-0-(2-hydroxyethyl)rapamycin (everolimus), or 40-O-[3-hydroxy-2-(hydroxymethyl)-2-methylpropanoate]rapamycin (temsirolimus).

As (A) rapamycin or a derivative thereof of the present invention, rapamycin (Sirolimus), everolimus, or temsirolimus is preferably used.

As such (A) rapamycin or a derivative thereof, a compound having a quality that can be sufficiently used as a pharmaceutical product is preferably used.

(B) The tocopherol derivative used in the present invention has been also known as vitamin E, and is widely and universally present in the nature as products such as olive oil, canola oil, soybean, almond, and blue-skin fish. (B) The tocopherol derivative is not particularly limited and any compound can be used herein, as long as it is a compound having a chroman-6-ol structure as a mother core. Examples of the tocopherol derivative that can be used herein include α-tocopherol, β-tocopherol, γ-tocopherol, δ-tocopherol, tocopherol acetate, α-tocotrienol, β-tocotrienol, γ-tocotrienol, and δ-tocotrienol. In addition, a D body, an L body, and a DL body are also present, and any one of them may be used herein.

In the present invention, as (B) the tocopherol derivative, the above-described tocopherol derivatives may be each used alone, or may also be used in combination.

(B) The tocopherol derivative used in the present invention is preferably α-tocopherol, and it is preferable to use D-α-tocopherol, L-α-tocopherol or DL-α-tocopherol.
(C) The ascorbic acid fatty acid ester according to the present invention is formed by binding fatty acid to ascorbic acid via an ester bond. (C) The ascorbic acid fatty acid ester is not particularly limited and any compound can be used herein, as long as it has a structure formed by introducing fatty acid into ascorbic acid via an ester bond.

An example of the fatty acid to be bound to ascorbic acid via an ester bond is monocarboxylic acid containing 2 to 30 carbon atoms (C2-C30). The fatty acid may be saturated fatty acid, or may also be unsaturated fatty acid containing one or more double bonds. Examples of the saturated fatty acid include acetic acid, propionic acid, butanoic acid, pentanoic acid, hexanoic acid, palmitic acid, stearic acid, eicosanoic acid, and docosanoic acid.
Examples of the unsaturated fatty acid include crotonic acid, myristoleic acid, palmitoleic acid, oleic acid, linoleic acid, and linolenic acid.
Examples of (C) the ascorbic acid fatty acid ester include L-ascorbyl stearate and ascorbyl palmitate.
In the present invention, as (C) the ascorbic acid fatty acid ester, the above-described ascorbic acid fatty acid esters may be each used alone, or may also be used in combination.
In the present invention, (B) the tocopherol derivative may be used in an amount of preferably 0.0001 part by mass or more, more preferably 0.0005 parts by mass or more, and further preferably 0.001 part by mass or more, based on 1 part by mass of (A) the rapamycin or a derivative thereof. In the present invention, since the tocopherol derivative is not particularly problematic in terms of safety, the upper limit of the use amount thereof should be determined, as appropriate, in an amount practically usable as a pharmaceutical product, while taking into consideration the stability of the rapamycin or a derivative thereof.
Taking into consideration the ensuring of the stability of the rapamycin or a derivative thereof and the realistic use amount of a pharmaceutical product additive, (B) the tocopherol derivative may be used in an amount of preferably 0.0001 to 5.0 parts by mass, more preferably 0.0005 to 1.0 part by mass, and further preferably 0.001 to 0.1 part by mass, based on 1 part by mass of (A) the rapamycin or a derivative thereof.
In the present invention, (C) the ascorbic acid fatty acid ester may be used in an amount of preferably 0.001 part by mass or more, more preferably 0.005 parts by mass or more, and further preferably 0.01 part by mass or more, based on 1 part by mass of (A) the rapamycin or a derivative thereof. In the present invention, since the ascorbic acid fatty acid ester does not have physical properties that impair the stability of the rapamycin or a derivative thereof, the upper limit of the use amount thereof is not particularly limited, and should be determined, as appropriate, in an amount practically usable as a pharmaceutical product.
Taking into consideration the ensuring of the stability of the rapamycin or a derivative thereof and the realistic use amount of a pharmaceutical product additive, (C) the ascorbic acid fatty acid ester may be used in an amount of preferably 0.001 to 10.0 parts by mass, more preferably 0.005 to 5.0 parts by mass, and further preferably 0.01 to 1.0 part by mass, based on 1 part by mass of (A) the rapamycin or a derivative thereof.
With regard to the mixing ratio of the components (B) and (C) in the present invention, the mass ratio between the components (B) and (C), (B) : (C), is preferably 1 : 0.1 to 100, and more preferably 1 : 0.5 to 100. (C) The ascorbic acid fatty acid ester is used, preferably at a mass ratio of equal to or greater than (B) the tocopherol derivative, and more preferably at a mass ratio (B) : (C) of 1 : 1 to 50.
The pharmaceutical composition of the present invention comprises three components, namely, (A) rapamycin or a derivative thereof, and (B) a tocopherol derivative and (C) an ascorbic acid fatty acid ester, and an aspect of mixing these components is not particularly limited and any mixing aspect is included in the present invention, as long as it is an aspect in which the components are mixed and are thus present. In general, (A) the rapamycin or a derivative thereof and (C) the ascorbic acid fatty acid ester are in the state of a solid at normal temperature, whereas (B) the tocopherol derivative is in the state of a liquid at normal temperature. As a method of mixing these components, a method, which comprises mixing the components (A) to (C) and mechanically blending them using any given mixer or the like, is applied. During such mixing operation, a suitable solvent may be added to the components, so that a dispersion of the components may be promoted. When the components (A) to (C) to be mixed are all solids, powdery or granular components having a small mean particle diameter of approximately 0.1 to 1 mm are advantageously used in terms of the improvement of dispersibility.

Alternatively, there may also be applied a method, which comprises preparing a solution containing any one or two of (A) the rapamycin or a derivative thereof, (B) the tocopherol derivative, and (C) the ascorbic acid fatty acid ester, then mixing the solution with the remaining component(s), and then arbitrarily mechanically blending them.

In the pharmaceutical composition of the present invention, the aspect, in which three components, namely, (A) the rapamycin or a derivative thereof, (B) the tocopherol derivative, and (C) the ascorbic acid fatty acid ester are mixed and are thus present, is preferably a solid mixture comprising (A) the rapamycin or a derivative thereof, (B) the tocopherol derivative, and (C) the ascorbic acid fatty acid ester, which is obtained by preparing a solution containing the three components and then removing the solvent from the solution. Such a mixture is considered to a mixture, in which the rapamycin or a derivative thereof is associated with the tocopherol derivative and the ascorbic acid fatty acid ester at a molecular level, and thus, this is a mixing form capable of exhibiting the highest stabilization effects of (B) the tocopherol derivative and (C) the ascorbic acid fatty acid ester on the rapamycin or a derivative thereof.

The solvent that can be used to prepare a solution containing (A) rapamycin or a derivative thereof, (B) the tocopherol derivative and (C) the ascorbic acid fatty acid ester is not limited, as long as both (A) rapamycin or a derivative thereof, (B) the tocopherol derivative and (C) the ascorbic acid fatty acid ester are dissolved therein. Examples of such a solvent include water, methanol, ethanol, 1-propanol, 2-propanol, 1-butanol, 2-butanol, 2-methyl-1-propanol, 3-methyl-1-butanol, 1-pentanol, ethylene glycol, glycerin, formic acid, acetic acid, acetone, methyl ethyl ketone, methyl isobutyl ketone, anisole, methyl acetate, ethyl acetate, ethyl formate, propyl acetate, isopropyl acetate, n-butyl acetate, isobutyl acetate, acetonitrile, diethyl ether, t-butyl methyl ether, tetrahydrofuran, 1,4-dioxane, diisopropyl ether, pentane, hexane, heptane, N,N-dimethylformamide, N,N-dimethylacetamide, N-methylpyrrolidone, 1,3-dimethyl-2-imidazolidinone, dimethyl sulfoxide, dichloromethane, and chloroform. These solvents may be used alone, or may also be used as a mixed solvent comprising two or more types of solvents. Examples of the solvent used herein are not limited thereto, but the aforementioned solvents can be used as applicable preferred solvents.

Taking into consideration the subsequent removal of a solvent, it is preferable to use a solvent having a boiling point of 120°C or lower, which can be distilled away under mild conditions. Examples of such a preferred solvent include water, methanol, ethanol, propanol, acetonitrile, acetone, methyl ethyl ketone, ethyl acetate, butyl acetate, tetrahydrofuran, 1,4-dioxane, pentane, heptane, diethyl ether, and t-butyl methyl ether.

The amount of the solvent used is not particularly limited, as long as (A) rapamycin or a derivative thereof, (B) the tocopherol derivative and (C) the ascorbic acid fatty acid ester are completely dissolved in the solvent. Hence, the amount of the solvent used can be adjusted, as appropriate.

In addition, upon preparation of the solution, the temperature is increased, as appropriate, so that dissolution of (A) rapamycin or a derivative thereof, (B) the tocopherol derivative and (C) the ascorbic acid fatty acid ester in the solvent may be promoted. The temperature of the solution upon preparation thereof is not particularly limited. Taking into consideration the stability of (A) rapamycin or a derivative thereof, it is preferable to prepare the solution at a temperature of 0°C to 80°C.

The method of preparing a solution containing rapamycin or a derivative thereof, tocopherol derivative and ascorbic acid fatty acid ester is not particularly limited in the present invention, as long as it is a method of dissolving the rapamycin or a derivative thereof, the tocopherol derivative and the ascorbic acid fatty acid ester in the solution. Examples of such a method include: a method, which comprises previously mixing rapamycin or a derivative thereof, tocopherol derivative and ascorbic acid fatty acid ester, then adding a solvent, in which the components are dissolved, to the mixture, and then dissolving the components in the solvent; and a method, which comprises mixing a solution prepared by adding a solvent to rapamycin or a derivative, with a solution prepared by adding a solvent to tocopherol derivative and ascorbic acid fatty acid ester. The method of preparing a solution containing rapamycin or a derivative thereof, tocopherol derivative and ascorbic acid fatty acid ester is not limited thereto, but these preparation methods can be used as applicable preferred preparation methods.

As a method of removing a solvent from a solution containing (A) rapamycin or a derivative thereof, (B) the tocopherol derivative and (C) the ascorbic acid fatty acid ester, a method of distilling the solvent away from the solution is applied. Regarding such a method of distilling the solvent away from the solution, the solvent can be removed from the solution by heating the solution. At that time, reduced pressure conditions are preferably applied because the solvent can be removed under mild temperature conditions. Otherwise, the solvent can also be removed by a spray-drying method, so as to obtain a solid mixture.

Moreover, there may also be adopted a method, which comprises precipitating a solid mixture comprising (A) rapamycin or a derivative thereof, (B) the tocopherol derivative and (C) the ascorbic acid fatty acid ester from a solution containing the components (A), (B) and (C), and then removing the solvent according to a filtration method. Examples of the method of precipitating a solid mixture include: what is called, a recrystallization method of promoting crystallization by cooling; and what is called, a precipitation method of adding a solvent for crystallization, which is miscible with a solution containing (A) rapamycin or a derivative thereof, (B) the tocopherol derivative and (C) the ascorbic acid fatty acid ester, and in which the components (A), (B) and (C) are insoluble or hardly-soluble, to the solid mixture, followed by crystallization.

The above-described solvent for crystallization is not particularly limited and any solvent can be applied herein, as long as it is miscible with a solution containing (A) rapamycin or a derivative thereof, (B) the tocopherol derivative and (C) the ascorbic acid fatty acid ester, and also, the components (A), (B) and (C) are insoluble or hardly soluble therein. Examples of the solvent for crystallization include water, diethyl ether, diisopropyl ether, tetrahydrofuran, 1,4-dioxane, hexane, heptane, and toluene. The type and use amount of the solvent for crystallization may be determined, as appropriate, depending on the type and amount of a solvent used in preparation of the solution.

A solvent for crystallization is added to a solution containing (A) rapamycin or a derivative thereof, (B) the tocopherol derivative and (C) the ascorbic acid fatty acid ester, so that a solid mixture containing the components (A), (B) and (C) is crystallized. After that, the mixture is arbitrarily cooled to promote crystallization, so as to prepare a suspension. Thereafter, the solvent is removed from the suspension by a filtration method, so as to obtain a solid mixture.

In the pharmaceutical composition of the present invention, as a stabilization adjuvant for ensuring the stability of the rapamycin or a derivative thereof, an antioxidant other than (B) the tocopherol derivative and (C) the ascorbic acid fatty acid ester, can be arbitrarily combined with (B) and (C), and can be then used.

As such an antioxidant, a known antioxidant exhibiting the effect of stabilizing the rapamycin and a derivative thereof can be used. Examples of such a known antioxidant include nitrite, ascorbic acid, sulfite, alpha-thioglycerin, edetic acid, erythorbic acid, cysteine hydrochloride, citric acid, dichloroisocyanuric acid, dibutylhydroxytoluene, lecithin, thioglycolic acid, thiomalic acid, pyrosulfite, butylhydroxyanisole, 1,3-butylene glycol, pentaerythritol tetrakis[3-(3,5-di-t-butyl-4-hydroxyphenyl)propionate]benzotriazole, isopropyl gallate, and 2-mercaptobenzimidazole. Examples of the antioxidant used herein are not limited thereto, but these compounds can be used as applicable preferred compounds.

The antioxidant can be used, as appropriate, in an amount that does not impair the stability of the rapamycin or a derivative thereof. When the antioxidant is used, the addition amount is not particularly limited. The antioxidant is preferably used in an amount of 0.0001 to 50 parts by mass, based on 1 part by mass of (A) the rapamycin or a derivative thereof. The antioxidant is used in an amount of more preferably 0.001 to 10 parts by mass, and further preferably 0.005 to 10.0 parts by mass.

The above-described other antioxidant is used by being added, separately, to a mixture of (A) the rapamycin or a derivative thereof, (B) the tocopherol derivative and (C) the ascorbic acid fatty acid ester. Alternatively, the antioxidant may be used by being added to a solid mixture prepared from the solution of the components (A), (B) and (C). Otherwise, a solution containing the components (A), (B) and (C) and an antioxidant is prepared, and the solvent is then removed from the solution, so that the antioxidant may be used in the form of a solid mixture comprising the components (A), (B) and (C) and the antioxidant.

When the antioxidant is used in the pharmaceutical composition of the present invention, it is preferable that a solution containing (A) the rapamycin or a derivative thereof, (B) the tocopherol derivative and (C) the ascorbic acid fatty acid ester and the other antioxidant be prepared, the solvent be removed from the solution, and the antioxidant be used in the form of a solid mixture comprising the components (A), (B) and (C) and the antioxidant. Examples of the method of removing the solvent, which is applied in the method of obtaining such a solid mixture, include a method involving distillation of the solvent and a method involving crystallization and filtration, as with the aforementioned methods. As such a solvent distillation method, a spray-drying method may also be used.

To the pharmaceutical composition of the present invention, a cellulose derivative and/or sugars may be added. These substances are used as pharmaceutical additives for preparing pharmaceutical product formulations.

The cellulose derivative is not particularly limited and any cellulose derivative can be used herein, as long as it is an additive that is commonly used in preparation of pharmaceutical product formulations. Examples of the cellulose derivative include crystalline cellulose, methyl cellulose, ethyl cellulose, cellulose acetate phthalate, low-substituted hydroxypropyl cellulose, hydroxypropyl cellulose, hydroxymethylpropyl cellulose, hydroxypropylmethyl cellulose acetate succinate, and hydroxypropylmethyl cellulose phthalate. Among these, it is preferable to use crystalline cellulose, methyl cellulose, ethyl cellulose, low-substituted hydroxypropyl cellulose, hydroxypropyl cellulose, and hydroxypropylmethyl cellulose.

Moreover, the sugars are not particularly limited and any sugars can be used herein, as long as they are additives that are commonly used in preparation of pharmaceutical product formulations. Examples of the sugars include arabinose, isomaltose, inositol, erythritol, galactosamine, galactose, xylitol, xylose, glucosamine, glucose, gentiobiose, kojibiose, sucrose, cellobiose, sophorose, sorbitol, thioglucose, turanose, deoxyribose, nigerose, palatinose, fucose, fructose, mannitol, maltose, mannose, melibiose, lactose, rhamnose, laminaribiose and trehalose. Among these, it is preferable to use lactose, mannitol, maltose, erythritol, sorbitol, fucose, xylitol, fructose, inositol, and trehalose.

The above-described cellulose derivative and/or sugars may be used alone, or in combination of multiple types.

The cellulose derivative and/or the sugars are used by being added to a pharmaceutical composition comprising (A) the rapamycin or a derivative thereof, (B) the tocopherol derivative and (C) the ascorbic acid fatty acid ester, and optionally given other antioxidant.

That is to say, the cellulose derivative and/or the sugars are used by being added to (A) the rapamycin or a derivative thereof, (B) the tocopherol derivative and (C) the ascorbic acid fatty acid ester, and optionally given other antioxidant, so as to prepare a mixture (addition method 1). Alternatively, the cellulose derivative and/or the sugars may be added to a solid mixture prepared from a solution containing the components (A), (B) and (C) and optionally given other antioxidant, so as to prepare a mixture (addition method 2).

When the cellulose derivative and/or sugars that are in a solid state are mixed with the components (A), (B) and (C) and optionally given other antioxidant that are also in a solid state, it is preferable that these components be mechanically mixed with one another using a mixer or the like, so that they are fully dispersed.

Furthermore, as an alternative method, a solution containing the components (A), (B) and (C) and optionally given antioxidant is mixed with the cellulose derivative and/or the sugars, and the solvent is then removed from this mixture, so that a mixture consisting of a solid mixture of the components (A), (B) and (C) and optionally given other antioxidant, and the cellulose derivative and/or the sugars, may be obtained and used (addition method 3).

The above-described addition method 3 is a method of adding the cellulose derivative and/or the sugars to a solution containing the components (A), (B) and (C) and optionally given other antioxidant. In this case, the cellulose derivative and/or the sugars are not necessarily dissolved in the solution, and they may be in the state of a suspension. The solvent is removed from this mixture, so that a mixture consisting of a solid mixture of the components (A), (B) and (C) and optionally given antioxidant, and the cellulose derivative and/or the sugars, can be prepared. As a method of removing the solvent, a method of distilling away the solvent is applied. The solvent is preferably distilled away under reduced pressure. Otherwise, a method of removing the solvent according to a spray-drying method may also be adopted.

The above-described solvent for crystallization is added to the solution to prepare a suspension, and the solvent is then removed by filtration, so that a mixture consisting of a solid mixture comprising the components (A), (B) and (C) and optionally given other antioxidant, and the cellulose derivative and/or the sugars, can be prepared.

In addition to the aforementioned (A) rapamycin or a derivative thereof, and (B) a tocopherol derivative and (C) an ascorbic acid fatty acid ester, as well as optionally given other antioxidant, cellulose derivative and sugars, the pharmaceutical composition of the present invention may also comprise other additives that are commonly used in preparation of pharmaceutical product formulations, in a range that does not impair the effects of the present invention. The present pharmaceutical composition may comprise, for example, an excipient, a disintegrator, a binder, a lubricant, a pH adjuster, inorganic salts, and a solvent.

Examples of the excipient include lactose, maltose, mannitol, erythritol, sorbitol, fucose, xylitol, fructose, inositol, and starch.

Examples of the disintegrator include carmellose, crospovidone, low-substituted hydroxypropyl cellulose, sodium starch glycolate, carmellose calcium, and croscarmellose sodium.

Examples of the binder include hydroxypropyl cellulose, hypromellose, polyvinyl alcohol, and polyvinyl pyrrolidone.

Examples of the lubricant include magnesium stearate, calcium stearate, sodium stearyl fumarate, talc, and sucrose fatty acid ester.

Examples of the pH adjuster include hydrochloric acid, sulfuric acid, phosphoric acid, citric acid, tartaric acid, malic acid, mesylic acid, tosylic acid, and besylic acid. A buffer, which comprises, as a main ingredient, such an acidic additive, and further, an alkaline metal salt, an alkaline-earth metal salt, or an ammonium salt, may also be used.

Examples of the inorganic salts include calcium chloride, sodium chloride, calcium oxide, and magnesium sulfate.

In general, examples of the solvent include water, a normal saline, a 5% glucose or mannitol aqueous solution, a water-soluble organic solvent (e.g., a single solvent such as glycerol, ethanol, dimethyl sulfoxide, N-methylpyrrolidone, polyethylene glycol, Cremophor, or a mixed solvent thereof), and polyethylene glycols (e.g., polyethylene glycol 300, polyethylene glycol 400, polyethylene glycol 600, polyethylene glycol 4000, etc.).

These additives can be used without any particular limitation, as long as they have purity that is acceptable for the intended use as pharmaceutical product formulations. These additives may be used alone, or may also be used as a mixture of the additives. These additives are optionally used, when the pharmaceutical composition or the pharmaceutical formulation is produced.

The pharmaceutical composition of the present invention can be produced in the form of a pharmaceutical product comprising the pharmaceutical composition.

Examples of the dosage form of this pharmaceutical product include: internal use agents, such as a tablet, a dispersible tablet, a chewable tablet, an effervescent tablet, a troche, a drop agent, a hard capsule, a soft capsule, a granule, a powder agent, a pill, dry syrup, infusions and/or decoctions, an electuary, syrup, a drink agent, a suspension, an orally disintegrating tablet, and a jelly agent; and external use agents, such as a suppository, a poultice, a plaster, an ointment, a cream agent, a mousse agent solution, a liquid agent, eye drops, an aerosol agent, and a spray agent. The dosage forms are not limited thereto, but these are applicable preferred dosage forms.

In a case where the pharmaceutical composition of the present invention is used in the form of an injection, examples of such an injection include an aqueous injection, a non-aqueous injection, a suspension injection, an emulsion injection, and also, as dosage forms of being dissolved or suspended at the time of use, an intradermal injection, a subcutaneous injection, an intramuscular injection, an intravenous injection, a central intravenous injection, an intra-arterial injection, and an intrathecal injection. Examples of the injection used herein are not limited thereto, but these injections can be used as applicable preferred dosage forms and administration routes.

The pharmaceutical product, in which the pharmaceutical composition of the present invention is used, can be applied to the treatment of disease. Examples of the disease, to which the pharmaceutical product can be applied include: transplant rejection in transplantation of heart, lung, combined heart-lung, liver, kidney, pancreas, skin, or cornea; autoimmune diseases, and inflammatory diseases, such as arthritis, rheumatic disease, systemic lupus erythematosus, multi polychondritis, crusts disease, Wegener's granulomatosis, dermatomyositis, chronic active hepatitis, myasthenia gravis, psoriasis, Steven-Johnson syndrome, idiopathic sprue, autoimmune inflammatory bowel disease, endocrine ophthalmopathy, Graves disease, nodule colitis, multiple sclerosis, primary biliary hepatitis, juvenile diabetes (type 1 diabetes), uveitis, keratoconjunctivitis sicca, vernal keratoconjunctivitis, interstitial lung fibrosis, psoriatic arthritis, glomerulonephritis, and juvenile dermatomyositis; asthma; and cancers and hyperproliferative diseases, such as breast cancer, renal cancer, neuroendocrine tumor, lymphoid proliferative disease, B cell lymphatic cancer, tuberous sclerosis, and proliferative skin disease. Examples of the disease are not limited thereto, but these diseases can be considered to be applicable preferred diseases.

The applied dose of the pharmaceutical product comprising the pharmaceutical composition of the present invention can be naturally changed, depending on the sex, age, physiological conditions, pathologic conditions of a patient, etc. For example, the rapamycin or a derivative thereof is administered to an adult patient at a daily dose of 0.01 to 100 mg/m$^2$ (body surface area). The dose of the pharmaceutical product is not limited thereto, but this dose can be used as an applicable preferred dose.

Examples

Hereinafter, the present invention will be more specifically described in the following examples. However, these examples are not intended to limit the scope of the present invention.

It is to be noted that, in the analysis using liquid chromatography (HPLC) in the present test examples, the measurement was carried out under the following conditions.

Measurement column: Zorbax Eclipse XDB-C18, Rapid resolution HT, 100 mm x 4.6 mm, 1.8 μm
Detector: ultraviolet absorption spectrophotometer (measurement wavelength: 278 nm)
Column temperature: 45°C
Mobile phase A: 0.1% formic acid; mobile phase B: methanol/acetonitrile = 50/50 Concentration gradient of mobile phase:

[Table 1]

| Time after injection (min) | Mobile phase A (vol %) | Mobile phase B (vol %) |
|---|---|---|
| 0 - 5 | 46 | 64 |
| 5 - 17 | 46 → 25 | 64 → 75 |
| 17 - 22 | 25 → 10 | 75 → 90 |
| 22 - 24 | 10 | 90 |
| 24 - 25 | 10 → 46 | 90 → 64 |
| 25 - 28 | 46 | 64 |

Flow rate: 1.5 mL/min

Injection amount: 10 μL

[Example 1]

50 mg of Everolimus was weighed into a test tube, and 90 μL of an ascorbyl palmitate (manufactured by Wako Pure Chemical Industries, Ltd.) EtOH solution (10 mg/mL) and 10 μL of a DL-α-tocopherol (Riken E Oil 1000, manufactured by RIKEN VITAMIN CO., LTD.) EtOH solution (10 mg/mL) were then added thereto. The mixture was irradiated with ultrasonic wave for 10 minutes, and thereafter, dissolution of everolimus was confirmed. After that, 100 μL of EtOH was added to the mixed solution to dilute it. Thereafter, 450 mg of anhydrous lactose (Super Tab (registered trademark) 21 AN, manufactured by DFE Pharma) and 50 mg of hypromellose (TC-5 E Type, manufactured by Shin-Etsu Chemical Co., Ltd.) were weighed into a mortar, and these substances were then stirred using a pestle to obtain a mixture. Thereafter, the above-obtained solution was added dropwise to this mixture, using a Pasteur pipette, and the thus obtained mixture was then stirred using a pestle. The obtained powders were transferred into an egg-plant shaped flask, and were then dried using an evaporator under reduced pressure for 3 hours, so as to produce the pharmaceutical composition of Example 1.

[Comparative Example 1]

70 mg of Everolimus was weighed into a test tube, and 200 μL of EtOH was then added thereto. The mixture was irradiated with ultrasonic wave for 10 minutes, and thereafter, dissolution of everolimus was confirmed. After that, 630 mg of anhydrous lactose (Super Tab (registered trademark) 21 AN, manufactured by DFE Pharma) and 70 mg of hypromellose (TC-5 E Type, manufactured by Shin-Etsu Chemical Co., Ltd.) were weighed into a mortar, and these substances were then stirred using a pestle to obtain a mixture. Thereafter, the above-obtained solution was added dropwise to this mixture, using a Pasteur pipette, and the thus obtained mixture was then stirred using a pestle. The obtained powders were transferred into an egg-plant shaped flask, and were then dried using an evaporator under reduced pressure for 3 hours.

[Comparative Example 2]

70 mg of Everolimus was weighed into a test tube, and 100 μL of a DL-α-tocopherol (Riken E Oil 1000, manufactured by RIKEN VITAMIN CO., LTD.) EtOH solution (14 mg/mL) was then added thereto. The mixture was irradiated with ultrasonic wave for 10 minutes, and thereafter, dissolution of everolimus was confirmed. After that, 100 μL of EtOH was added to the mixed solution to dilute it. Thereafter, 630 mg of anhydrous lactose (Super Tab (registered trademark) 21 AN, manufactured by DFE Pharma) and 70 mg of hypromellose (TC-5 E Type, manufactured by Shin-Etsu Chemical Co., Ltd.) were weighed into a mortar, and these substances were then stirred using a pestle to obtain a mixture. Thereafter, the above-obtained solution was added dropwise to this mixture, using a Pasteur pipette, and the thus obtained mixture was then stirred using a pestle. The obtained powders were transferred into an egg-plant shaped flask, and were then dried using an evaporator under reduced pressure for 3 hours.

[Comparative Example 3]

70 mg of Everolimus was weighed into a test tube, and 100 μL of an ascorbyl palmitate (manufactured by Wako Pure Chemical Industries, Ltd.) EtOH solution (14 mg/mL) was then added thereto. The mixture was irradiated with ultrasonic wave for 10 minutes, and thereafter, dissolution of everolimus was confirmed. After that, 100 μL of EtOH was added to the mixed solution to dilute it. Thereafter, 630 mg of anhydrous lactose (Super Tab (registered trademark) 21 AN, manufactured by DFE Pharma) and 70 mg of hypromellose (TC-5 E Type, manufactured by Shin-Etsu Chemical Co., Ltd.) were weighed into a mortar, and these substances were then stirred using a pestle to obtain a mixture. Thereafter, the above-obtained solution was added dropwise to this mixture, using a Pasteur pipette, and the thus obtained mixture was then stirred using a pestle. The obtained powders were transferred into an egg-plant shaped flask, and were then dried using an evaporator under reduced pressure for 3 hours.

[Test Example 1]

The pharmaceutical compositions obtained by the methods applied in Example 1 and Comparative Examples 1 to 3 (approximately 500 mg each) were each collected in a brown sample bottle, and were then preserved under light-shielded conditions at 60°C, in a desiccator, the humidity of which had been adjusted with a saturated cobalt chloride aqueous solution, while the bottle was uncapped.

The residual amounts of everolimus 3 days and 14 days after initiation of the preservation were measured by liquid chromatography (HPLC), and the residual percentages of everolimus at individual time points were then calculated. It is to be noted that the residual percentage was calculated according to the following formula. The results are shown in Table 2.

Residual percentage (%) of everolimus = (peak area of everolimus measured by HPLC at each time point/weighed value of powders) / (peak area of everolimus measured by HPLC before preservation (initial)/weighed value of powders) x 100

[Table 2]

| | Additive | Residual percentage (%) of everolimus | |
|---|---|---|---|
| | | Value on Day 3 | Value on Day 14 |
| Example 1 | DL-α-tocopherol + ascorbyl palmitate | 96 | 90 |
| Comparative Example 1 | - | 88 | 28 |
| Comparative Example 2 | DL-α-tocopherol | 85 | 35 |
| Comparative Example 3 | Ascorbyl palmitate | 90 | 28 |

As a result, when DL-α-tocopherol or ascorbyl palmitate was used alone as an antioxidant in Comparative Examples 2 and 3, decomposition of everolimus progressed at the same level as in the case of Comparative Example 1 in which no antioxidants were added, and thus, it became clear that DL-α-tocopherol or ascorbyl palmitate does not exhibit antioxidative effects on a compound having a rapamycin structure as a mother core. However, surprisingly, it became clear that these antioxidants, which do not exhibit antioxidative effects when used alone, exhibit strong everolimus-stabilizing effects by being combined with each other.

**Claims**

1. A pharmaceutical composition comprising (A) rapamycin or a derivative thereof, and (B) a tocopherol derivative and (C) an ascorbic acid fatty acid ester.

2. The pharmaceutical composition according to claim 1, which is produced by preparing a solution containing (A) rapamycin or a derivative thereof, and (B) a tocopherol derivative and (C) an ascorbic acid fatty acid ester, and then removing the solvent from the solution.

3. The pharmaceutical composition according to claim 1 or 2, which comprises 0.0001 to 5.0 parts by mass of (B) the tocopherol derivative and 0.001 to 10.0 parts by mass of (C) the ascorbic acid fatty acid ester, based on 1 part by mass of (A) the rapamycin or a derivative thereof, and the mixing ratio between the components (B) and (C), (B) : (C), is 1 : 0.1 to 100.

4. The pharmaceutical composition according to any one of claims 1 to 3, which further comprises an antioxidant other than (B) the tocopherol derivative and (C) the ascorbic acid fatty acid ester.

5. The pharmaceutical composition according to any one of claim 1 to 4, which comprises a cellulose derivative and/or sugars.

6. A pharmaceutical formulation comprising the pharmaceutical composition according to any one of claims 1 to 5.

7. A method for producing a pharmaceutical composition comprising rapamycin or a derivative thereof, which comprises preparing a solution containing (A) rapamycin or a derivative thereof, and (B) a tocopherol derivative and (C) an ascorbic acid fatty acid ester, and then removing the solvent from the solution.

8. A method for producing a pharmaceutical formulation comprising rapamycin or a derivative thereof, which comprises preparing a solution containing (A) rapamycin or a derivative thereof, and (B) a tocopherol derivative and (C) an ascorbic acid fatty acid ester, then mixing the solution with a cellulose derivative and/or sugars, and then removing the solvent from the mixture.

9. A method for producing a pharmaceutical formulation comprising rapamycin or a derivative thereof, which comprises preparing a solution containing (A) rapamycin or a derivative thereof, and (B) a tocopherol derivative and (C) an ascorbic acid fatty acid ester, and then removing the solvent from the prepared solution to obtain a pharmaceutical composition comprising the rapamycin or a derivative thereof, and adding a cellulose derivative and/or sugars to said pharmaceutical composition.

## INTERNATIONAL SEARCH REPORT

| International application No. |
|---|
| PCT/JP2016/074781 |

| A. CLASSIFICATION OF SUBJECT MATTER |
|---|
| *A61K31/436*(2006.01)i, *A61P29/00*(2006.01)i, *A61P35/00*(2006.01)i, *A61P37/02* (2006.01)i, *A61P37/06*(2006.01)i |

According to International Patent Classification (IPC) or to both national classification and IPC

| B. FIELDS SEARCHED |
|---|
| Minimum documentation searched (classification system followed by classification symbols) |
| A61K31/436, A61P29/00, A61P35/00, A61P37/02, A61P37/06 |

| Documentation searched other than minimum documentation to the extent that such documents are included in the fields searched |
|---|
| Jitsuyo Shinan Koho       1922-1996   Jitsuyo Shinan Toroku Koho   1996-2016 |
| Kokai Jitsuyo Shinan Koho   1971-2016   Toroku Jitsuyo Shinan Koho   1994-2016 |

| Electronic data base consulted during the international search (name of data base and, where practicable, search terms used) |
|---|
| JSTPlus/JMEDPlus/JST7580(JDreamIII), |
| CAplus/REGISTRY/MEDLINE/EMBASE/BIOSIS(STN) |

| C. DOCUMENTS CONSIDERED TO BE RELEVANT |
|---|

| Category* | Citation of document, with indication, where appropriate, of the relevant passages | Relevant to claim No. |
|---|---|---|
| X | CN 102499929 A  (Fujian Institute of Microbiology), 20 June 2012 (20.06.2012), example 10 (Family: none) | 1,5,8 |
| X | CN 102319207 A  (Fujian Institute of Microbiology), 18 January 2012 (18.01.2012), example 3 (Family: none) | 1,3 |

| [X] Further documents are listed in the continuation of Box C. | [ ] See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 30 September 2016 (30.09.16) | 11 October 2016 (11.10.16) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

**INTERNATIONAL SEARCH REPORT**

International application No.

PCT/JP2016/074781

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2002-531527 A  (Novartis AG.),<br>24 September 2002 (24.09.2002),<br>claim 1; paragraphs [0010], [0013], [0015] to [0021]<br>& WO 2000/033878 A2<br>claim 1; page 2, lines 10 to 20; page 3, lines 2 to 3; page 3, line 7 to page 4, line 18<br>& US 2002/0032213 A1      & GB 9826882 A<br>& EP 1137439 A2            & DE 69936352 D<br>& FR 2786771 A             & BR 9915986 A<br>& CA 2351580 A             & KR 10-2006-0096477 A<br>& CN 1374872 A | 1-9 |
| Y | JP 2006-510753 A  (Council of Scientific and Industrial Research),<br>30 March 2006 (30.03.2006),<br>paragraph [0003]<br>& WO 2004/054391 A1<br>page 1, 4th line from the bottom to page 2, line 1<br>& US 2009/0081322 A1      & US 2006/0165826 A1<br>& EP 1571929 A1            & CA 2510300 A<br>& CN 1735355 A | 1-9 |
| Y | JP 3-56585 A  (Shokuhin Sangyo High Separation System Gijutsu Kenkyu Kumiai),<br>12 March 1991 (12.03.1991),<br>page 2, upper left column, lines 2 to 3<br>(Family: none) | 1-9 |
| Y | JP 63-130503 A  (Nitto Electric Industrial Co., Ltd.),<br>02 June 1988 (02.06.1988),<br>page 2, upper left column, lines 1 to 3<br>(Family: none) | 1-9 |
| Y | JP 10-505359 A  (Novo Nordisk A/S),<br>26 May 1998 (26.05.1998),<br>example 11<br>& WO 1996/034606 A1<br>example 11<br>& US 5866590 A            & EP 830132 A1<br>& BR 9608120 A            & CA 2220019 A<br>& CN 1183721 A            & KR 10-0440354 B | 1-9 |
| Y | Kwi-Hyun HA et al., "Disappearance and interrelationship of tocopherol analogues during autoxidation of corn oil, and synergistic effect of L-ascorbyl palmitate with ALPHA-tocopherol", Nihon Shokuhin Kogyo Gakkaishi, 1988, vol.35, no.7, pages 464 to 470, Abstract,'3 Results and Study', paragraph of '2. α-Toc to L-AsA palmitate tono Sojo Koka' | 1-9 |

Form PCT/ISA/210 (continuation of second sheet) (January 2015)

**EP 3 342 410 A1**

**INTERNATIONAL SEARCH REPORT**

International application No.

PCT/JP2016/074781

C (Continuation).   DOCUMENTS CONSIDERED TO BE RELEVANT

| Category* | Citation of document, with indication, where appropriate, of the relevant passages | Relevant to claim No. |
|---|---|---|
| Y | Minoru AOYAMA et al., "Studies on the Improvement of Antioxidant Effect of Tocopherols. XXIII. Synergistic Effect of L-Ascorbyl Palmitate", Journal of the Japan Oil Chemists' Society, 1993, vol.42, no.1, pages 25 to 29, Abstract, '3 Results and Study', paragraph of '3.1 Oven Shiken ni yoru AP to Toc tono Sojosei' | 1-9 |
| Y | JP 11-509223 A  (Novartis AG.), 17 August 1999 (17.08.1999), example 1 & WO 1997/03654 A1 example 1 & US 6004973 A         & GB 9514397 A & EP 839028 A2        & FR 2736550 A & BR 9609537 A        & RU 2159107 C & CN 1195289 A        & KR 10-0352943 B | 1-9 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11509223 A
- JP 2005507897 A
- JP 2002531527 A
- WO 2013022201 A
- WO 94022136 A
- US 5258389 A
- WO 9409010 A
- WO 9205179 A
- US 5118677 A
- US 5100883 A
- US 5151413 A